# EUROPEAN PATENT SPECIFICATION

(11) **EP 2 268 321 B1**
(45) Date of publication and mention of the grant of the patent: **09.10.2013**
(21) Application number: 09738213.9
(22) Date of filing: 30.04.2009
(51) Int. Cl.: A61K 49/10, G01N 33/50, C12Q 1/52

(54) **Method of determining alanine transaminase (ALT) activity by 13C-MR detection using hyperpolarised 13C-pyruvate**
Verfahren zur Bestimmung einer Alanintransaminase-Aktivität durch 13C-MR-Detektion mit Hilfe von hyperpolarisiertem 13C-Pyruvat
Procédé de détermination de l'activité de la transaminase alanine (ALT) par détection 13C-MR au moyen de 13C-pyruvate hyperpolarisé

(30) Priority: 02.05.2008 US 49795
(43) Date of publication of application: 05.01.2011
(73) Proprietor: General Electric Company, Schenectady, NY 12345 (US); The Regents of The University of California, Oakland, CA 94607 (US)
(72) Inventor: HU, Zhong-Min, McKinleyville California 95519 (US); HURD, Ralph, Eugene, Milpitas California 95035 (US); KURHANEWICZ, John, South San Francisco California 94080 (US); NELSON, Sarah, Jane, Belmont California 94002 (US); VIGNERON, Daniel, Blackburn, Corte Madera California 94925 (US)
(74) Representative: Livgard, Kim Are Birkeli
(86) International application number: PCT/EP2009/055258
(87) International publication number: WO 2009/133169

(56) References cited:
- WO-A2-92/01937
- WO-A2-2005/078438
- WO-A2-2005/113761
- US-A1- 2007 196 280
- GOLMAN K ET AL: "Real-time metabolic imaging" PROCEEDINGS OF THE NATIONAL ACADEMY OF SCIENCES OF USA, NATIONAL ACADEMY OF SCIENCE, WASHINGTON, DC., US, vol. 103, no. 30, 25 July 2006 (2006-07-25), pages 11270-11275, XP002460340 ISSN: 0027-8424
- CHANCE E M ET AL: "MATHEMATICAL ANALYSIS OF ISOTOPE LABELING IN THE CITRIC-ACID CYCLE WITH APPLICATIONS TO CARBON-13 NMR STUDIES IN PERFUSED RAT HEARTS" JOURNAL OF BIOLOGICAL CHEMISTRY, vol. 258, no. 22, 1983, pages 13785-13794, XP002536924 ISSN: 0021-9258
- CARVALHO RUI A ET AL: "Hepatic gluconeogenesis and Krebs cycle fluxes in a CCl4 model of acute liver failure." NMR IN BIOMEDICINE FEB 2002, vol. 15, no. 1, February 2002 (2002-02), pages 45-51, XP002536925 ISSN: 0952-3480
- MERRITT MATTHEW E ET AL: "Inhibition of Carbohydrate Oxidation During the First Minute of Reperfusion After Brief Ischemia: NMR Detection of Hyperpolarized (CO2)-C-13 and (HCO3-)-C-13" MAGNETIC RESONANCE IN MEDICINE, vol. 60, no. 5, November 2008 (2008-11), pages 1029-1036, XP002536926 ISSN: 0740-3194
- 'Glucose-Alanine Cycle', [Online] Retrieved from the Internet: <URL:http://pathman.smpdb.ca/pathways/SMP00 127/pathway> [retrieved on 2012-02-27]

## Description

The invention relates to an *in vitro* method of determination of alanine transaminase (ALT) activity by ¹³C-MR detection using an imaging medium which comprises hyperpolarised ¹³C-pyruvate. Further, the invention relates to an imaging medium comprising hyperpolarised ¹³C-pyruvate for use in a method of determining ALT activity by *in vivo* ¹³C-MR.

ALT, also known as glutamate pyruvate transaminase (GPT) and alanine aminotransferase (ALAT) is an enzyme that catalyzes the reversible transamination between alanine and α-ketoglutarate to form pyruvate and glutamate. By mediating the conversion of these four major metabolites, ALT plays an important role in gluconeogenesis and amino acid metabolism. In muscle and certain other tissues, ALT degrades amino acids for fuel, and amino groups are collected from glutamate by transamination. ALT transfers α-amino group from glutamate to pyruvate to form alanine, which is a major amino acid in blood during fasting. Alanine is taken up by the liver for generating glucose from pyruvate in a reverse ALT reaction, constituting the so-called alanine-glucose cycle. This cycle is also important during intensive exercise when skeletal muscles operate anaerobically, producing not only ammonia groups from protein breakdown but also large amounts of pyruvate from glycolysis.

Perhaps the most well-known aspect of ALT is that it is used clinically as an index of liver integrity or hepatocellular damage. Serum ALT activity is significantly elevated in a variety of liver damage conditions including viral infection, alcoholic steatosis, nonalcoholic steatohepatitis (NASH), and drug toxicity. While low level of ALT is present in peripheral circulation because of normal cell turnover or release from nonvascular sources, the liver has been shown to contain the highest levels of ALT. The difference between ALT levels in liver and in blood has been shown to be about 2,000-3,000-fold. Hence, the increased ALT in serum, plasma, or blood is regarded as a marker of liver injury because of the "leakage" of hepatic ALT into the circulation. Usually, the nature of liver injury causes the blood ALT levels to vary greatly. Extremely high transaminase levels (greater than 8- to 10-fold normal) can indicate acute viral hepatitis and/or drug-induced hepatotoxicity. A mild chronic increase of serum ALT (2- to 8-fold) is generally a characteristic of chronic hepatitis, fatty liver, and/or steatosis. However, many details of the mechanism for the correlation of ALT levels with the etiology of liver damage remain to be understood.

Even though serum ALT is one of the most widely-used assays in clinical chemistry, there are serious deficiencies with the assay because it is an inadequate predictor in some cases. Recent studies have cast doubt on serum ALT assay's specificity for liver disease. Higher than normal ALT levels are frequently associated with other clinical conditions such as obesity, muscle disease, heart failure, hemochromatosis, Wilson's disease, or antitrypsin deficiency.

There is a need for improved methods for determining ALT activity that more directly and accurately indicate and/or diagnose liver tissue injury and/or disease. Further, there is a need for improved methods for determining the ALT activity in assessing response to treatment of liver tissue injuries and/or diseases, e.g. response to lifestyle modifications or treatment with drugs.

Various methods for the determination of ALT activity are known which mostly rely on determining serum ALT.

Serum ALT activity is typically measured *in vitro* by the continuous monitoring of pyruvate produced by the enzyme's reaction. This is accomplished by a coupled enzymatic reaction using lactate dehydrogenase to catalytically reducing pyruvate to lactate with the concurrent oxidation of reduced nicotinamide adenine dinucleotide (NADH) to its oxidized form, NAD. This reaction is measured spectrophotometrically by following the decrease in the absorbance (usually at 340 nm) which is due to the oxidation of NADH. An IFCC recommended formulation exists for serum ALT activity determination.

WO-A-2005/113761 discloses ALT polypeptides and antibodies that specifically bind to said polypeptides which can be used in diagnosing or detecting injury or disease involving tissue which contains said ALT polypeptides. Samples of bodily fluids from an animal or patients are used in said *in vitro* diagnosis or detection.

US 5,705,045 discloses a biosensor capable of measuring ALT and AST (aspartate transaminase) activity. The biosensor consists of two sets of electrodes which are sensitive to ALT and AST, respectively. In an assay employing this biosensor, a biological fluid like serum or plasma containing ALT and/or AST is placed on the biosensor.

However all the ALT determination methods described above use blood samples as a basis and hence it is not sure that when elevated ALT levels have been determined, these are due to liver injuries or diseases. Hence there is a need for new and improved methods to determine ALT activity, especially ALT activity localized directly to the liver.

PNAS vol.103, no. 30, 25 July 2006, pages 11270-11275 teaches the use of hyperpolarised ¹³C-pyruvate, obtained by DNP, for real-time metabolic mapping of its metabolites.

It has now been found that hyperpolarised ¹³C-pyruvate can be used as an agent for determining ALT activity *in vivo,* for instance directly in the liver and *in vitro* by using ¹³C-MR detection.

As described above ALT catalyzes the reversible reaction between hyperpolarised ¹³C-pyruvate and glutamate to form hyperpolarised ¹³C-alanine and α-ketoglutarate. It has been found that an increased ALT activity in livers of fasted rats - a model for assessing liver metabolic state - manifests itself in a low ¹³C-alanine signal compared to livers of non-fasted rats, while the ¹³C-lactate signal remained unchanged. The decreased hyperpolarized ¹³C-alanine levels observed in fasted rat liver point to a shift in the ALT-mediated ¹³C-pyruvate/¹³C-alanine reaction equilibrium, i.e. a decrease in ¹³C-alanine due to heightened ALT levels. During fasting, ALT levels in rats have been shown to increase, promoting the use of alanine as a gluconeogenic substrate and its conversion to pyruvate for eventual glucose generation (F. Rosen et al., J. Bio. Chem. 234(3), 1958, 476-480). The decrease in hyperpolarized ¹³C-alanine detected might also be due to decreased endogenous alanine in the fasted liver, i.e. lower starting alanine, which would affect the final hyperpolarized ¹³C-alanine equilibrium.

The ability to detect altered ALT activity/altered alanine metabolism in the liver might be useful for studying and identifying liver diseases such as hepatitis, fatty liver and cirrhosis and for monitoring therapy of liver diseases.

It has been found and described earlier that the metabolic conversion of hyperpolarised ¹³C-pyruvate into its metabolites hyperpolarised ¹³C-lactate, hyperpolarised ¹³C-bicarbonate (in the case of ¹³C₁-pyruvate, ¹³C_{1,2}-pyruvate or ¹³C_{1,2,3}-pyruvate only) and hyperpolarised ¹³C-alanine can be used to study metabolic processes in the human and non-human animal body using ¹³C-MR. ¹³C₁-pyravate has a T₁ relaxation in human full blood at 37° C of about 42 s, however, the conversion of hyperpolarised ¹³C-pyruvate to hyperpolarised ¹³C-lactate, hyperpolarised ¹³C-bicarbonate and hyperpolarised ¹³C-alanine has been found to be fast enough to allow signal detection from the ¹³C-pyruvate parent compound and its metabolites. The amount of alanine, bicarbonate and lactate is dependent on the metabolic status of the tissue under investigation. The MR signal intensity of hyperpolarised ¹³C-lactate, hyperpolarised ¹³C-bicarbonate and hyperpolarised ¹³C-alanine is related to the amount of these compounds and the degree of polarisation left at the time of detection, hence by monitoring the conversion of hyperpolarised ¹³C-pyruvate to hyperpolarised ¹³C-lactate, hyperpolarised ¹³C-bicarbonate and hyperpolarised ¹³C-alanine it is possible to study metabolic processes *in vivo* in the human or non-human animal body by using non-invasive MR imaging or MR spectroscopy.

It has further been found that the MR signal amplitudes arising from the different pyruvate metabolites varies depending on the tissue type. The unique metabolic peak pattern formed by alanine, lactate, bicarbonate and pyruvate can be used as fingerprint for the metabolic state of the tissue under examination and thus allows for the discrimination between healthy tissue and tumour tissue. The use of hyperpolarised ¹³C-pyruvate for tumour imaging - with tumour tissue showing high metabolic activity - has been described in detail in WO-A-2006/011810.

Further, the use of hyperpolarised ¹³C-pyruvate for cardiac imaging has been described in WO-A-2006/054903.

Thus, in a first aspect the invention provides a method for monitoring progression of a liver disease, determining the severity of a liver disease or identifying patients at risk to develop a liver disease and/or candidates for preventive measures to avoid the development of an acute or chronic liver disease by using an imaging medium comprising hyperpolarised ¹³C-pyruvate and determining ALT activity localised directly to the liver by *in vitro* ¹³C-MR detection wherein the signal of ¹³C-alanine and optionally ¹³C-lactate and/or ¹³C-pyruvate is detected, and wherein the signal of ¹³C-alanine and optionally ¹³C-lactate and/or ¹³C-pyruvate is used to generate a metabolic profile indicative for the ALT activity of the liver cells or *ex vivo* tissue from the liver biopsy or from the isolated liver under examination.

A second aspect of the invention relates to an imaging medium comprising hyperpolarised ¹³C-pyruvate for use in a method of determining ALT activity localised directly to the liver by *in vivo* ¹³C-MR detection wherein the signal of ¹³C-alanine and optionally ¹³C-lactate and/or ¹³C-pyruvate is detected, and wherein the signal of ¹³C-alanine and optionally ¹³C-lactate and/or ¹³C-pyruvate is used to generate a metabolic profile indicative for the ALT activity of the liver under examination, wherein the information obtained by the metabolic profile is used for monitoring progression of a liver disease, determining the severity of a liver disease or identifying patients at risk to develop a liver disease and/or candidates for preventive measures to avoid the development of an acute or chronic liver disease.

The term "determining ALT activity" denotes the initial measurement of ALT activity by measuring the dynamics and/or maximum conversion of ¹³C-pyruvate to ¹³C-alanine through the ALT enzyme.

The term ¹³C-MR detection" denotes ¹³C-MR imaging or ¹³C-MR spectroscopy or combined ¹³C-MR imaging and ¹³C-MR spectroscopy, i.e. ¹³C-MR spectroscopic imaging. The term further denotes ¹³C-MR spectroscopic imaging at various time points.

The term "imaging medium" denotes a liquid composition comprising hyperpolarised ¹³C-pyruvate as the MR active agent, i.e. imaging agent.

The imaging medium may be used as an imaging medium for *in vivo* ¹³C-MR detection, i.e. in living human or non-human animal beings. Further, the imaging medium may be used as imaging medium for *in vitro* ¹³C-MR detection, e.g. in cell cultures, body samples such as blood, *ex vivo* tissue, for instance *ex vivo* tissue obtained from a biopsy or isolated organs derived from an animal or human body.

The term "¹³C-pyruvate" denotes a salt of ¹³C-pyruvic acid that is isotopically enriched with ¹³C, i.e. in which the amount of ¹³C isotope is greater than its natural abundance.

The isotopic enrichment of the hyperpolarised ¹³C-pyruvate used in the method of the invention is preferably at least 75%, more preferably at least 80% and especially preferably at least 90%, an isotopic enrichment of over 90% being most preferred. Ideally, the enrichment is 100%. ¹³C-pyruvate in said imaging medium used in the method of the invention may be isotopically enriched at the C1-position (in the following denoted ¹³C₁-pyruvate), at the C2-position (in the following denoted ¹3C₂-pyruvate), at the C3-position (in the following denoted ¹³C₃-pyruvate), at the C1- and the C2-position (in the following denoted ¹³C_{1,2}-pyruvate), at the C1- and the C3-position (in the following denoted ¹³C_{1,3}-pyruvate), at the C2- and the C3-position (in the following denoted ¹³C_{2,3}-pyruvate) or at the C1-, C2- and C3-position (in the following denoted ¹³C_{1,2,3}-pyruvate). Isotopic enrichment at the C1-position is preferred since ¹³C₁-pyruvate has a higher T₁ relaxation in human full blood at 37° C (about 42 s) than ¹³C-pyruvate which is isotopically enriched at other C-positions.

The terms "hyperpolarised" and "polarised" are used interchangeably hereinafter and denote a nuclear polarisation level in excess of 0.1%, more preferred in excess of 1% and most preferred in excess of 10%.

The level of polarisation may for instance be determined by solid state ¹³C-NMR measurements in solid hyperpolarised ¹³C-pyruvate, e.g. solid hyperpolarised ¹³C-pyruvate obtained by dynamic nuclear polarisation (DNP) of ¹³C-pyruvate. The solid state ¹³C-NMR measurement preferably consists of a simple pulse-acquire NMR sequence using a low flip angle. The signal intensity of the hyperpolarised ¹³C-pyruvate in the NMR spectrum is compared with signal intensity of ¹³C-pyruvate in a NMR spectrum acquired before the polarisation process. The level of polarisation is then calculated from the ratio of the signal intensities of before and after polarisation.

In a similar way, the level of polarisation for dissolved hyperpolarised ¹³C-pyruvate may be determined by liquid state NMR measurements. Again the signal intensity of the dissolved hyperpolarised ¹³C-pyruvate is compared with the signal intensity of a reference sample of known composition, e.g. liquid pyruvic acid or sodium pyruvate dissolved in an aqueous solution. The level of polarisation is then calculated from the ratio of the signal integrals of hyperpolarised ¹³C-pyruvate and the known reference sample, optionally corrected for the relative concentrations. The polarisation can also be determined by comparing with the thermal equilibrium signal of the same ¹³C-pyruvate sample after the hyperpolarisation has died away.

Hyperpolarisation of NMR active ¹³C-nuclei may be achieved by different methods which are for instance described in WO-A-98/30918, WO-A-99/24080 and WO-A-99/35508 and hyperpolarisation methods are polarisation transfer from a noble gas, "brute force", spin refrigeration, the parahydrogen method and dynamic nuclear polarisation (DNP).

To obtain hyperpolarised ¹³C-pyurvate, it is preferred to either polarise ¹³C-pyruvate directly or to polarise ¹³C-pyruvic acid and convert the polarised ¹³C-pyruvic acid to polarised ¹³C-pyruvate, e.g. by neutralisation with a base.

One suitable way for obtaining hyperpolarised ¹³C-pyruvate is the polarisation transfer from a hyperpolarised noble gas which is described in WO-A-98/30918. Noble gases having non-zero nuclear spin can be hyperpolarised by the use of circularly polarised light. A hyperpolarised noble gas, preferably He or Xe, or a mixture of such gases, may be used to effect hyperpolarisation of ¹³C-nuclei. The hyperpolarised gas may be in the gas phase, it may be dissolved in a liquid/solvent, or the hyperpolarised gas itself may serve as a solvent. Alternatively, the gas may be condensed onto a cooled solid surface and used in this form, or allowed to sublime. Intimate mixing of the hyperpolarised gas with ¹³C-pyruvate or ¹³C-pyruvic acid is preferred. Hence, if ¹³C-pyruvic acid is polarised, which is a liquid at room temperature, the hyperpolarised gas is preferably dissolved in a liquid/solvent or serves as a solvent. If ¹³C pyruvate is polarised, the hyperpolarised gas is preferably dissolved in a liquid/solvent, which also dissolves pyruvate.

Another suitable way for obtaining hyperpolarised ¹³C-pyruvate is that polarisation is imparted to ¹³C-nuclei by thermodynamic equilibration at a very low temperature and high field. Hyperpolarisation compared to the operating field and temperature of the NMR spectrometer is effected by use of a very high field and very low temperature (brute force). The magnetic field strength used should be as high as possible, suitably higher than 1 T, preferably higher than 5 T, more preferably 15 T or more and especially preferably 20 T or more. The temperature should be very low, e.g. 4.2 K or less, preferably 1.5 K or less, more preferably 1.0 K or less, especially preferably 100 mK or less.

Another suitable way for obtaining hyperpolarised ¹³C-pyruvate is the spin refrigeration method. This method covers spin polarisation of a solid compound or system by spin refrigeration polarisation. The system is doped with or intimately mixed with suitable crystalline paramagnetic materials such as Ni²⁺, lanthanide or actinide ions with a symmetry axis of order three or more. The instrumentation is simpler than required for DNP with no need for a uniform magnetic field since no resonance excitation field is applied. The process is carried out by physically rotating the sample around an axis perpendicular to the direction of the magnetic field. The pre-requisite for this method is that the paramagnetic species has a highly anisotropic g-factor. As a result of the sample rotation, the electron paramagnetic resonance will be brought in contact with the nuclear spins, leading to a decrease in the nuclear spin temperature. Sample rotation is carried out until the nuclear spin polarisation has reached a new equilibrium.

In a preferred embodiment, DNP (dynamic nuclear polarisation) is used to obtain hyperpolarised ¹³C-pyruvate. In DNP, polarisation of MR active nuclei in a compound to be polarized is affected by a polarisation agent or so-called DNP agent, a compound comprising unpaired electrons. During the DNP process, energy, normally in the form of microwave radiation, is provided, which will initially excite the DNP agent. Upon decay to the ground state, there is a transfer of polarisation from the unpaired electron of the DNP agent to the NMR active nuclei of the compound to be polarised, e.g. to the ¹³C nuclei in ¹³C-pyruvate. Generally, a moderate or high magnetic field and a very low temperature are used in the DNP process, e.g. by carrying out the DNP process in liquid helium and a magnetic field of about 1 T or above. Alternatively, a moderate magnetic field and any temperature at which sufficient polarisation enhancement is achieved may be employed. The DNP technique is for example further described in WO-A-98/58272 and in WO-A-01/96895.

To polarise a compound by the DNP method, a mixture of the compound to be polarised and a DNP agent is prepared ("a sample") which is either frozen and inserted as a solid into a DNP polariser for polarisation or which is inserted into a DNP polariser as a liquid and freezes inside said polariser due to the very low surrounding temperature. After the polarisation, the frozen solid hyperpolarised sample is rapidly transferred into the liquid state either by melting it or by dissolving it in a suitable dissolution medium. Dissolution is preferred and the dissolution process of a frozen hyperpolarised sample and suitable devices therefore are described in detail in WO-A-02/37132. The melting process and suitable devices for the melting are for instance described in WO-A-02/36005.

In order to obtain a high polarisation level in the compound to be polarised said compound and the DNP agent need to be in intimate contact during the DNP process. This is not the case if the sample crystallizes upon being frozen or cooled. To avoid crystallization, either glass formers need to be present in the sample or compounds need to be chosen for polarisation which do not crystallize upon being frozen but rather form a glass.

As mentioned earlier ¹³C-pyruvic acid or ¹³C-pyruvate are suitable starting materials to obtain hyperpolarized ¹³C-pyruvate.

Isotopically enriched ¹³C-pyruvate is commercially available, e.g. as sodium ¹³C-pyruvate. Alternatively, it may be synthesized as described by S. Anker, J. Biol. Chem 176, 1948, 133-1335.

Several methods for the synthesis of ¹³C₁-pyruvic acid are known in the art. Briefly, Seebach et al., Journal of Organic Chemistry 40(2), 1975, 231-237 describe a synthetic route that relies on the protection and activation of a carbonyl-containing starting material as an S,S-acetal, e.g. 1,3-dithian or 2-methyl-1,3-dithian. The dithiane is metallated and reacted with a methyl-containing compound and/or ¹³CO₂. By using the appropriate isotopically enriched ¹³C-component as outlined in this reference, it is possible to obtain ¹³C₁-pyruvate or ¹³C_{1,2}-pyruvate. The carbonyl function is subsequently liberated by use of conventional methods described in the literature. A different synthetic route starts from acetic acid, which is first converted into acetyl bromide and then reacted with Cu¹³CN. The nitrile obtained is converted into pyruvic acid via the amide (see for instance S.H. Anker et al., J. Biol. Chem. 176 (1948), 1333 or J. E. Thirkettle, Chem Commun. (1997), 1025). Further, ¹³C-pyruvic acid may be obtained by protonating commercially available sodium ¹³C-pyruvate, e.g. by the method described in US 6,232,497 or by the method described in WO-A-2006/038811.

The hyperpolarisation of ¹³C-pyruvic acid by DNP is described in detail in WO-A1-2006/011809. Briefly, ¹³C-pyruvic acid may be directly used for DNP since it forms a glass when frozen. After DNP, the frozen hyperpolarised ¹³C-pyruvic acid needs to be dissolved and neutralised, i.e. converted to ¹³C-pyruvate. For the conversion, a strong base is needed. Further, since ¹³C-pyruvic acid is a strong acid, a DNP agent needs to be chosen which is stable in this strong acid. A preferred base is sodium hydroxide and conversion of hyperpolarised ¹³C-pyruvic acid with sodium hydroxide results in hyperpolarised sodium ¹³C-pyruvate, which is the preferred ¹³C-pyruvate for an imaging medium which is used for *in vivo* MR imaging and/or spectroscopy, i.e. MR imaging and/or spectroscopy carried out on living human or non-human animal beings.

Alternatively, ¹³C-pyruvate, i.e. a salt of ¹³C-pyruvic acid can be used for DNP. Preferred salts are those ¹³C-pyruvates which comprise an inorganic cation from the group consisting of NH₄⁺, K⁺, Rb⁺, Cs⁺, Ca²⁺, Sr²⁺ and Ba²⁺, preferably NH₄⁺, K⁺, Rb⁺ or Cs⁺, more preferably K⁺, Rb⁺, Cs⁺ and most preferably Cs⁺, as in detail described in WO-A-2007/111515. The synthesis of these preferred ¹³C-pyruvates is disclosed in WO-A-2007/111515 as well. If the hyperpolarized ¹³C-pyruvate is used in an imaging medium for *in vivo* MR imaging and/or spectroscopy it is preferred to exchange the inorganic cation from the group consisting of NH₄⁺, K⁺, Rb⁺, Cs⁺, Ca²⁺, Sr²⁺ and Ba²⁺ by a physiologically very well tolerable cation like Na⁺ or meglumine. This may be done by methods known in the art like the use of a cation exchange column.

Further preferred salts are ¹³C-pyruvates of an organic amine or amino compound, preferably TRIS-¹³C₁-pyruvate or meglumine-¹³C₁-pyruvate, as in detail described in WO-A2-2007/069909. The synthesis of these preferred ¹³C-pyruvates is disclosed in WO-A2-2007/069909 as well.

If the hyperpolarised ¹³C-pyruvate is obtained by DNP, the sample to be polarised comprising ¹³C-pyruvic acid or ¹³C-pyruvate and a DNP agent may further comprise a paramagnetic metal ion. The presence of paramagnetic metal ions in composition to be polarised by DNP has found to result in increased polarisation levels in the ¹³C-pyruvic acid/¹³C-pyruvate as described in detail in WO-A2-2007/064226.

As mentioned earlier, the imaging medium may be used as imaging medium for *in vivo* ALT activity determination by ¹³C-MR detection, i.e. in living human or non-human animal beings. For this purpose, the imaging medium is provided as a composition that is suitable for being administered to a living human or non-human animal body. Such an imaging medium preferably comprises in addition to the MR active agent ¹³C-pyruvate an aqueous carrier, preferably a physiologically tolerable and pharmaceutically accepted aqueous carrier like water, a buffer solution or saline. Such an imaging medium may further comprise conventional pharmaceutical or veterinary carriers or excipients, e.g. formulation aids such as are conventional for diagnostic compositions in human or veterinary medicine.

Further, the imaging medium may be used as imaging medium for *in vitro* ALT activity determination by ¹³C-MR detection, i.e. in cell cultures, body samples such as blood samples, *ex vivo* tissues such as biopsy tissue or isolated organs. For this purpose, the imaging medium is provided as a composition that is suitable for being added to, for instance, cell cultures, blood samples, *ex vivo* tissues like biopsy tissue or isolated organs. Such an imaging medium preferably comprises in addition to the MR active agent ¹³C-pyruvate a solvent which is compatible with and used for *in vitro* cell or tissue assays, for instance DMSO or methanol or solvent mixtures comprising an aqueous carrier and a non aqueous solvent, for instance mixtures of DMSO and water or a buffer solution or methanol and water or a buffer solution. As it is apparent for the skilled person, pharmaceutically acceptable carriers, excipients and formulation aids may be present in such an imaging medium but are not required for such a purpose.

If the imaging medium is used for *in vivo* determination of ALT activity, i.e. in a living human or non-human animal body, said imaging medium is preferably administered to said body parenterally, preferably intravenously. Generally, the body under examination is positioned in an MR magnet. Dedicated ¹³C-MR RF-coils are positioned to cover the area of interest. Exact dosage and concentration of the imaging medium will depend upon a range of factors such as toxicity and the administration route. Suitably, the imaging medium is administered in a concentration of up to 1 mmol pyruvate per kg bodyweight, preferably 0.01 to 0.5 mmol/kg, more preferably 0.1 to 0.3 mmol/kg. At less than 400 s after the administration, preferably less than 120 s, more preferably less than 60 s after the administration, especially preferably 20 to 50 s an MR imaging sequence is applied that encodes the volume of interest in a combined frequency and spatial selective way. The exact time of applying an MR sequence is highly dependent on the volume of interest.

If the imaging medium is used for *in vitro* determination of ALT activity, said imaging medium is 1 mM to 100 mM in ¹³C-pyruvate, more preferably 20 mM to 90 mM and most preferably 40 to 80 mM in ¹³C-pyruvate.

ALT activity can be determined by detecting the ¹³C-alanine signal and optionally the ¹³C-lactate and/or ¹³C-pyruvate signal. The determination is based on the following reaction which is illustrated for ¹³C₁-pyruvate; * denotes the ¹³C-label: 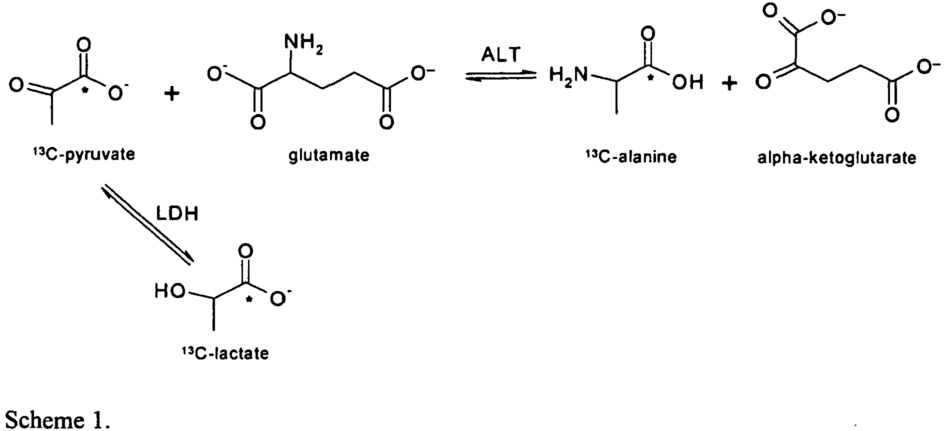 According to scheme 1, ¹³C-pyruvate and glutamate react in a reversible reaction catalyzed by ALT to form ¹³C-alanine and α-ketoglutarate. In another reversible reaction ¹³C-pyruvate is converted to ¹³C-lactate. As described earlier we have found that an increased ALT activity manifests itself in a low ¹³C-alanine signal.

The term "signal" in the context of the invention refers to the MR signal amplitude or integral or peak area to noise of peaks in a ¹³C-MR spectrum which represent ¹³C-alanine and optionally ¹³C-lactate and/or ¹³C-pyruvate. In a preferred embodiment, the signal is the peak area.

In a preferred embodiment, the signals of ¹³C-alanine and ¹³C-lactate are detected.

In a preferred embodiment, the signals of ¹³C-alanine and ¹³C-lactate are used to generate said metabolic profile.

In one embodiment, the spectral signal intensity of ¹³C-alanine and optionally ¹³C-lactate and/or ¹³C-pyruvate is used to generate the metabolic profile. In another embodiment, the spectral signal integral of ¹³C-alanine and optionally ¹³C-lactate and/or ¹³C-pyruvate is used to generate the metabolic profile. In another embodiment, signal intensities from separate images of ¹³C-alanine and optionally ¹³C-lactate and/or ¹³C-pyruvate are used to generate the metabolic profile. In yet another embodiment, the signal intensities of ¹³C-alanine and optionally ¹³C-lactate and/or ¹³C-pyruvate are obtained at two or more time points to calculate the rate of change of ¹³C-alanine and optionally ¹³C-lactate and/or ¹³C-pyruvate.

In another embodiment the metabolic profile includes or is generated using processed signal data of ¹³C-alanine and optionally ¹³C-lactate and/or ¹³C-pyruvate, e.g. ratios of signals, corrected signals, or dynamic or metabolic rate constant information deduced from the signal pattern of multiple MR detections, i.e. spectra or images. Thus, in a preferred embodiment a corrected ¹³C-alanine signal, i.e. ¹³C-alanine to ¹³C-lactate and/or ¹³C-alanine to ¹³C-pyruvate signal is included into or used to generate the metabolic profile. In a further preferred embodiment, a corrected ¹³C-alanine to total ¹³C-carbon signal is included into or used to generate the metabolic profile with total ¹³C-carbon signal being the sum of the signals of ¹³C-alanine and ¹³C-lactate and/or ¹³C-pyruvate. In a more preferred embodiment, the ratio of ¹³C-alanine to ¹³C-lactate and/or ¹³C-pyruvate is included into or used to generate the metabolic profile.

Early treatment (e.g. changes in lifestyle) of liver related diseases like for instance non-viral hepatitis prevents some of the most devastating complications connected to such liver diseases, like for instance chronic hepatitis or liver cirrhosis. Optimal approaches for identifying patients at risk and/or candidates for preventive measures like lifestyle changes involving control of diabetes and hyperlipidemia, weight loss in overweight patients and abstinence from alcohol remain to be determined. It would thus be beneficial to have a method which is useful to identify patients at risk to develop liver diseases and to identify candidates for preventive measures. The present invention may provide the necessary information to make that identification. In this embodiment, the method of the invention may be used to determine the initial ALT activity at a first time point and to make subsequent ALT activity determinations over a period of time at a certain frequency, e.g. semi-annually or annually. It can be expected that an increase in ALT activity will indicate an increasing risk to develop liver diseases and rate of increase can be used by the physician to decide on commencement of preventive measures and/or treatment. Further, the results of the determination of ALT activity over time could be combined with results from other liver function tests like AST or ALP determination and the combined results may be used to make a decision on preventive measures and/or treatment. To carry out the method of the invention for the above-mentioned purpose *in vitro* does of course require that suitable samples from a patient under treatment are obtainable. Alternatively, *in vivo* ¹³C-MR detection results in detection of ALT activity directly in the liver imaging and hence the information obtained may be directly and conveniently be used for identifying patients at risk to develop a liver disease and/or candidates for preventive measures to avoid the development of an acute or chronic liver disease.

In yet another embodiment the information obtained is used to monitor progression of a liver disease. This may be useful for liver diseases or liver disorders where the disease has not progressed to a level where treatment is indicated or recommended, e.g. because of severe side-effects associated with said treatment. In such a situation the choice of action is a close monitoring of the patient for disease progression and early detection of deterioration. In this embodiment, the invention may be used to determine the initial ALT activity and to make subsequent ALT activity determinations over a period of time at a certain frequency. For liver diseases, it can be expected that an increase in ALT activity will indicate progress and worsening of the disease and the said increase can be used by the physician to decide on commencement of treatment. To carry out the method of the invention for the above-mentioned purpose *in vitro* does of course require that suitable samples from a patient under treatment are obtainable.

In yet another embodiment the information obtained is used for determining the severity of a liver disease. Often diseases progress from their onset over time. Depending on the kind of symptoms and/or the finding of certain clinical markers diseases are characterized by certain stages, e.g. an early (mild) stage, a middle (moderate) stage and a severe (late) stage. More refined stages are common for certain diseases. A variety of clinical markers is known to be used for staging a disease including more specific ones like certain enzymes or protein expression but also more general ones like blood values, electrolyte levels etc. In this context, ALT activity may be such a clinical marker which is used - alone or in combination with other markers and/or symptoms - to determine a disease stage and thus severity of a disease. Hence it may be possible to use the invention for determining ALT activity in the liver in a patient in a quantitative way and from the ALT activity value obtained staging the patient's disease. ALT ranges which are characteristic for a certain disease stage may be established by determining ALT activity according to the invention in patients having for instance a disease in an early, middle and late stage and defining a range of ALT activity which is characteristic for a certain stage.

Since ALT activity is influenced by a variety of factors like dietary status or exercise it is important to control these factors, e.g. by providing patients with a diet plan or standardized meals prior to carrying out the method of the invention. Also, it has been found that the patient is not fasted since this would result in a decreased ¹³C-alanine signal.

Anatomical and/or - where suitable - perfusion information may be included in the method of the invention when carried out *in vivo.* Anatomical information may for instance be obtained by acquiring a proton or ¹³C-MR image with or without employing a suitable contrast agent before or after the method of the invention.

### Brief description of the drawings:

FIG. 1 shows features in dynamic curves (FIG. 1a) and in a single voxel spectrum (FIG. 1b) from a 3D-¹³C-MR spectroscopic imaging acquisition.

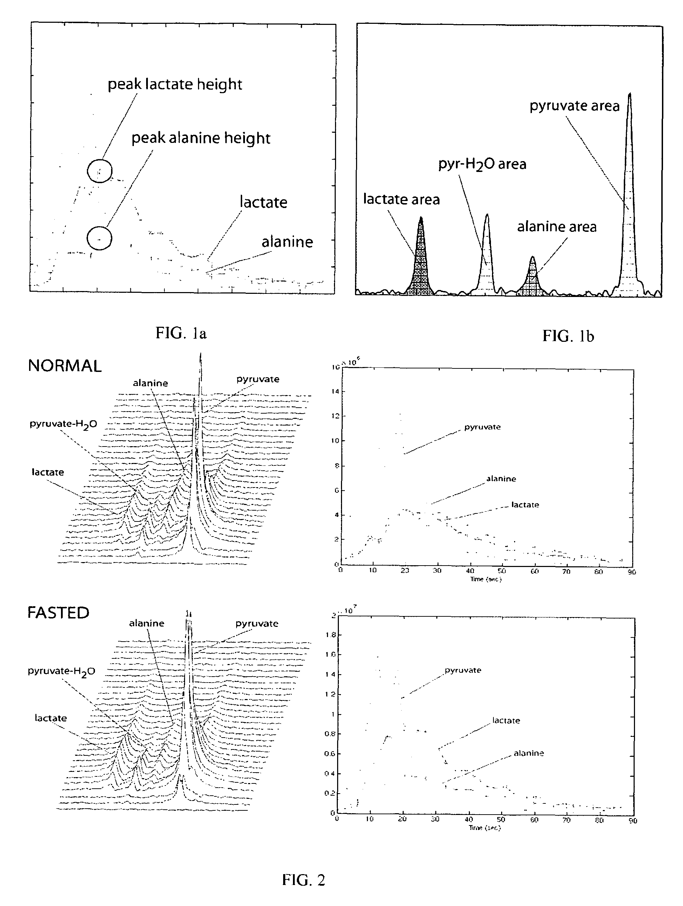

FIG. 2 shows representative liver slice localized dynamic curves from normal and fasted rats. These final dynamic curves were derived from the stack plot insets in which each horizontal line in a stack plot represents a separate magnitude spectrum of the hyperpolarized species collected every 3 seconds. For the sake of clarity, the pyruvate-hydrate has been omitted from the final plotted dynamic curves, and the pyruvate curve has been scaled down by a factor of four for easier viewing. In the dynamic curves, each marked point represents the intensity of ¹³C-pyruvate (∼171 ppm), ¹³C-lactate (∼183 ppm), and ¹³C-alanine (∼176 ppm) at that time point, i.e. a trace of those ridges in the associated stack plot, showing the uptake and conversion of ¹³C-pyruvate.
FIG. 3 shows all data points for peak ¹³C-lactate/¹³C-alanine ratio from normal and fasted rat ¹³C-MR spectroscopy slice acquisitions. Triangular markers show the collected data points and the square marker/error bars show the mean/standard errors.
FIG. 4 shows the comparison of representative liver slice spectra from 3D-¹³C-MR spectroscopic imaging spectra with 1 cm³ voxel resolution of normal (FIG. 4a) and fasted (FIG. 4b) rats. Typically the rat liver spanned a couple of slices.
FIG. 5 shows ¹³C-lactate to total carbon fraction (FIG. 5a) and ¹³C-alanine to total carbon fraction (FIG. 5b) from 3D-¹³C-MR spectroscopic imaging studies (averaged over liver voxels per rat) of normal and fasted rat livers. Triangular markers show the collected data points and the square marker/error bars show the mean ± standard errors. Note that five normal ¹³C-alanine to total carbon points overlap, thus obscuring the bottom two points.

### Examples

In the following the terms pyruvate, ¹³C-pyruvate and ¹³C₁-pyruvate are used interchangeably and all denote ¹³C₁-pyruvate. The terms pyruvic acid, ¹³C-pyruvic acid and ¹³C₁₋pyruvic acid are used interchangeably and all denote ¹³C₁-pyruvic acid. The terms alanine, ¹³C-alanine and ¹³C₁-alanine are used interchangeably and all denote ¹³C₁-alanine. The terms lactate, ¹³C-lactate and ¹³C₁-lactate are used interchangeably and all denote ¹³C₁-lactate.

### Example 1 Production of an imaging medium comprising hyperpolarised ¹³C₁-pyruvate obtained by the DNP method

Tris(8-carboxy-2,2,6,6-(tetra(hydroxyethyl)-benzo-[1,2-4,5']-bis-(1,3)-dithiole-4-yl)-methyl sodium salt (trityl radical) which had been synthesised according to Example 7 of WO-A1-98/39277 was added to ¹³C-pyruvic acid (40 mM) in a test tube to result in a composition being 15mM in trityl radical.

The composition was transferred from the test tube to a sample cup and the sample cup was inserted into a HyperSense^{™} DNP polariser (Oxford Instruments). The composition was polarised under DNP conditions at 1.4 °K in a 3.35 T magnetic field under irradiation with microwave (93.89 GHz) for 45 min.

The composition was subsequently dissolved in an aqueous solution of sodium hydroxide, TRIS buffer and EDTA at a pressure of 10 bar and temperature of 170 °C. The resultant imaging medium contained 80 mM of hyperpolarized sodium ¹³C₁-pyruvate at pH 7.2 - 7.9, with a polarization of about 18% during administration.

### Example 2 Fasted rat liver models - animal preparation

Two groups of rats were included in this study, to investigate liver metabolism both in fasted and non-fasted rats. Non-fasted rates were allowed to feed freely while fasted rats had their food removed about 24 hrs before MR-detection.

### Example 3 ¹³C-MR detection

### Example 3a Animal preparation

A catheter was introduced into the tail vein, and rats were then placed in MR scanner.

### Example 3b Hyperpolarised ¹³C-pyruvate dosing and administration

3 ml of the imaging medium as prepared in Example 1 was injected over 12 s via the tail vein catheter into the rat.

### Example 3c ¹³C-MR imaging/spectroscopy

A home-built dual tuned ¹H/¹³C RF coil was fit over the rat abdomen, localising signal from the liver. Rats were positioned in a 3 T horizontal bore GE MR scanner.

For the ¹³C-MR spectroscopy experiments, a slice selective (15mm slab select centered on the liver) RF pulse with 5° flip angle was applied every 3 s starting with the injection. The collected data, processed using MATLAB, were apodized with a 10 Hz Lorentzian filter before Fourier transformation, and the dynamic data points were taken from magnitude spectra. From the processed dynamic curves, peak ¹³C-lactate height and peak ¹³C-alanine height were used to derive peak ¹³C-lactate to ¹³C-alanine ratio used for statistical comparisons (see FIG. 1).

For the 3D-¹³C-MR spectroscopic imaging experiments, acquisitions were performed using a double-spinecho sequence (Cunningham et al., J. Mag. Reson. 187:357-362 (2007) with variable flip angle, centric phase encoding order, TE = 140 ms, TR = 215 ms (total acquisition time of 14 s), FOV = 8 x 8 cm, and 1 cc resolution. From the processed 3D magnitude spectra, for each rat, the voxels containing mostly liver tissue as seen from the anatomical images were manually labeled. For each liver voxel, the area under the ¹³C-pyruvate, ¹³C-pyruvate-hydrate, ¹³C-lactate, and ¹³C-alanine peaks in the magnitude spectra were calculated, with the sum of these four areas termed total carbon area (see FIG. 1). Lactate area to total carbon area and alanine area to total carbon area were calculated for each voxel and then averaged over all liver voxels to derive the test statistics average lactate to total carbon ratio and average alanine to total carbon ratio.

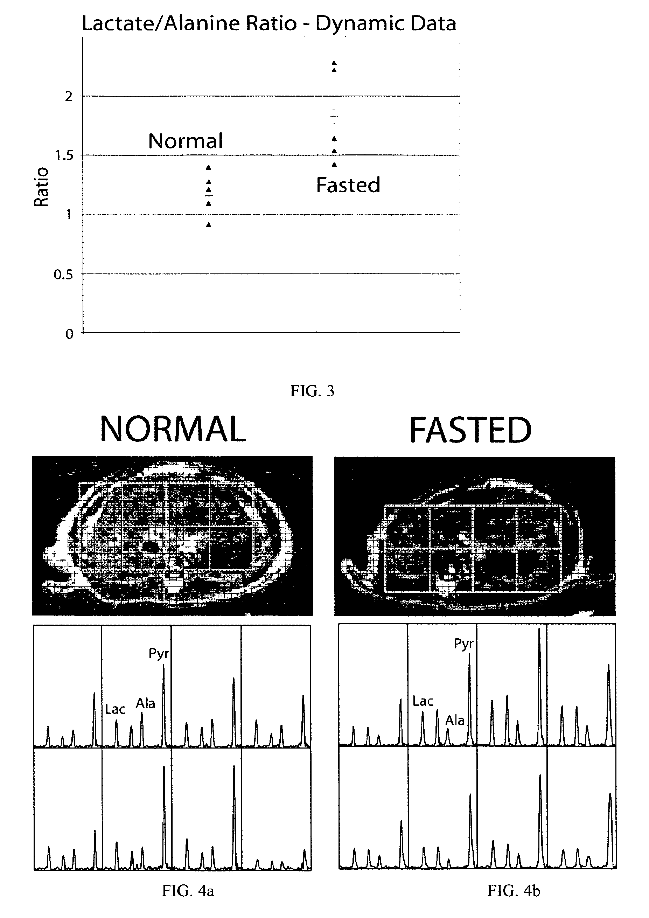

FIG. 2 shows representative liver slice localized dynamic curves from normal and fasted rats. These final dynamic curves were derived from the stack plot insets in which each horizontal line in a stack plot represents a separate magnitude spectrum of the hyperpolarized species collected every 3 seconds. For the sake of clarity, the pyruvate-hydrate has been omitted from the final plotted dynamic curves, and the pyruvate curve has been scaled down by a factor of four for easier viewing. In the dynamic curves, each marked point represents the intensity of pyruvate (∼171 ppm), lactate (∼183 ppm), and alanine (∼176 ppm) at that time point, i.e. a trace of those ridges in the associated stack plot, showing the uptake and conversion of pyruvate. Typically, the lactate and alanine curves plateaued around 20-30 seconds after injection, meaning the highest lactate and alanine SNR occurred in this range. This is important for picking an imaging window for the 3D-¹³C-MR spectroscopic imaging acquisitions, in which the SNR from each voxel is much lower than that in the whole slice MRS experiments. Qualitatively, the lactate and alanine curves in the normal rats had similar maximum amplitudes while there was a dramatic difference in the fasted rats (see FIG. 3). Using a Mann-Whitney Rank-Sum test, there was a statistically significant difference in lactate-to-alanine ratio (P < 0.01).

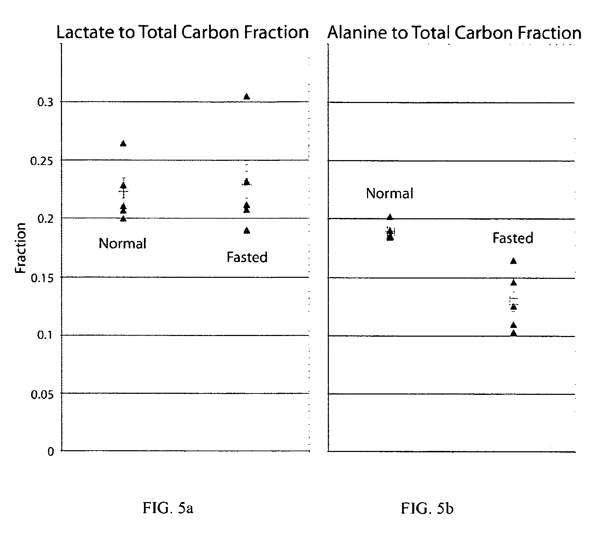

FIG. 4 shows representative slices from 3D-MRSI spectra with 1 cm³ voxel resolution of normal and fasted rat liver (typically the rat liver spanned a couple of slices). All the fasted liver voxel spectra showed a high lactate-to-alanine ratio, corroborating what was seen in the MRS acquisitions. Qualitatively, the lactate levels looked comparable between the normal and fasted liver spectra, but alanine was lower in the latter. The average lactate area to total carbon area and average alanine area to total carbon area ratios were calculated for each rat. FIG. 5 shows these lactate fractions. Using a Mann-Whitney Rank-Sum test, there was no statistically significant difference in lactate to total carbon area between normal and fasted groups (P = 0.42), but there was a statistically significant difference in alanine to total carbon area between normal and fasted groups (P < 0.01).

## Claims

1. A method for monitoring progression of a liver disease, determining the severity of a liver disease or identifying patients at risk to develop a liver disease and/or candidates for preventive measures to avoid the development of an acute or chronic liver disease by using an imaging medium comprising hyperpolarised ¹³C-pyruvate and determining ALT activity localised directly to the liver by *in vitro* ¹³C-MR detection wherein the signal of ¹³C-alanine and optionally ¹³C-lactate and/or ¹³C-pyruvate is detected, and wherein the signal of ¹³C-alanine and optionally ¹³C-lactate and/or ¹³C-pyruvate is used to generate a metabolic profile indicative for the ALT activity of the liver cells or *ex vivo* tissue from the liver biopsy or from the isolated liver under examination.

2. The method as claimed in claim 1, wherein the ALT activity is determined at a first time point and at subsequent time point over a period of time.

3. The method as claimed in claim 1, wherein the hyperpolarized ¹³C-pyruvate is obtained by dynamic nuclear polarization of ¹³C-pyruvic acid or ¹³C-pyruvate.

4. An imaging medium comprising hyperpolarised ¹³C-pyruvate for use in a method of determining ALT activity localised directly to the liver by *in vivo* ¹³C-MR detection wherein the signal of ¹³C-alanine and optionally ¹³C-lactate and/or ¹³C-pyruvate is detected, and wherein the signal of ¹³C-alanine and optionally ¹³C-lactate and/or ¹³C-pyruvate is used to generate a metabolic profile indicative for the ALT activity of the liver under examination, wherein the information obtained by the metabolic profile is used for monitoring progression of a liver disease, determining the severity of a liver disease or identifying patients at risk to develop a liver disease and/or candidates for preventive measures to avoid the development of an acute or chronic liver disease.

5. The imaging medium for use as claimed in claim 4, wherein the ALT activity is determined at a first time point and at subsequent time point over a period of time.

6. The imaging medium for use as claimed in claim 4, wherein the imaging medium is administered to a human or non-human animal body and an MR imaging sequence is applied at less than 400 seconds after administration

7. The imaging medium for use as claimed in claim 4, wherein the hyperpolarized ¹³C-pyruvate is obtained by dynamic nuclear polarization of ¹³C-pyruvic acid or ¹³C-pyruvate.

## Patentansprüche

1. Verfahren zur Überwachung des Fortschreitens einer Lebererkrankung, Bestimmung der Schwere einer Lebererkrankung oder Identifizierung von Patienten mit Risiko dafür, eine Lebererkrankung zu entwickeln und/oder von Kandidaten für Vorbeugungsmaßnahmen, um die Entwicklung einer akuten oder chronischen Lebererkrankung unter Verwendung eines Bildgebungsmittels umfassend hyperpolarisiertes ¹³C-Pyruvat und Bestimmung der auf der Leber direkt lokalisierten ALT-Aktivität durch *in vitro*-¹³C-MR-Detektion zu vermeiden, wobei das Signal von ¹³C-Alanin und wahlweise ¹³C-Lactat und/oder ¹³C-Pyruvat detektiert wird, und wobei das Signal von ¹³C-Alanin und wahlweise ¹³C-Lactat und/oder ¹³C-Pyruvat dazu verwendet wird, ein metabolisches Profil zu erzeugen, welches für die ALT-Aktivität der Leberzellen oder des *ex vivo*-Gewebes aus der Leberbiopsie oder aus der untersuchten isolierten Leber indikativ ist.

2. Verfahren nach Anspruch 1, wobei die ALT-Aktivität zu einem ersten Zeitpunkt und zu einem nachfolgenden Zeitpunkt während einer Zeitperiode bestimmt wird.

3. Verfahren nach Anspruch 1, wobei das hyperpolarisierte ¹³C-Pyruvat durch dynamische Kernpolarisierung von ¹³C-Brenztraubensäure oder ¹³C-Pyruvat erhalten wird.

4. Bildgebungsmittel umfassend hyperpolarisiertes ¹³C-Pyruvat zur Verwendung bei einem Verfahren zur Bestimmung der auf der Leber direkt lokalisierten ALT-Aktivität durch *in vitro*-¹³C-MR-Detektion, wobei das Signal von ¹³C-Alanin und wahlweise ¹³C-Lactat und/oder ¹³C-Pyruvat detektiert wird, und wobei das Signal von ¹³C-Alanin und wahlweise ¹³C-Lactat und/oder ¹³C-Pyruvat dazu verwendet wird, ein metabolisches Profil zu erzeugen, welches für die ALT-Aktivität der untersuchten Leber indikativ ist, wobei die durch das metabolische Profil erhaltene Information zur Überwachung des Fortschreitens einer Lebererkrankung, Bestimmung der Schwere einer Lebererkrankung oder Identifizierung von Patienten mit Risiko dafür, eine Lebererkrankung zu entwickeln und/oder von Kandidaten für Vorbeugungsmaßnahmen verwendet wird, um die Entwicklung einer akuten oder chronischen Lebererkrankung zu vermeiden.

5. Bildgebungsmittel zur Verwendung nach Anspruch 4, wobei die ALT-Aktivität zu einem ersten Zeitpunkt und zu einem nachfolgenden Zeitpunkt während einer Zeitperiode bestimmt wird.

6. Bildgebungsmittel zur Verwendung nach Anspruch 4, wobei das Bildgebungsmittel in einem menschlichen oder nicht-menschlichen Tierkörper verabreicht wird, und eine MR-Bildgebungssequenz in weniger als 400 Sekunden nach der Verabreichung verwendet wird.

7. Bildgebungsmittel zur Verwendung nach Anspruch 4, wobei das hyperpolarisierte ¹³C-Pyruvat durch dynamische Kernpolarisierung von ¹³C-Brenztraubensäure oder ¹³C-Pyruvat erhalten wird.

## Revendications

1. Procédé de surveillance de la progression d'une maladie du foie, de détermination de la gravité d'une maladie du foie ou d'identification de patients à risque de développer une maladie du foie et/ou de candidats de mesures préventives pour éviter le développement d'une maladie du foie aigué ou chronique en utilisant un milieu d'imagerie comprenant du pyruvate ¹³C hyperpolarisé et de détermination d'activité d'ALT localisée directement au foie par la détection RM-¹³C *in vitro,* le signal de l'alanine ¹³C et éventuellement du lactate ¹³C et/ou du pyruvate ¹³C étant détecté, et le signal de l'alanine ¹³C et éventuellement du lactate ¹³C et/ou du pyruvate ¹³C étant utilisé pour générer un profil métabolique indicatif de l'activité d'ALT des cellules du foie ou du tissu *ex vivo* issu de la biopsie du foie ou du foie isolé en cours d'examen.

2. Procédé selon la revendication 1, dans lequel l'activité d'ALT est déterminée à un premier instant et à un instant ultérieur au cours d'une période de temps.

3. Procédé selon la revendication 1, dans lequel le pyruvate ¹³C hyperpolarisé est obtenu par une polarisation nucléaire dynamique de l'acide pyruvique ¹³C ou du pyruvate ¹³C.

4. Milieu d'imagerie comprenant le pyruvate ¹³C hyperpolarisé à utiliser dans un procédé de détermination d'activité d'ALT localisée directement au foie par la détection RM-¹³C *in vitro,* le signal de l'alanine ¹³C et éventuellement du lactate ¹³C et/ou du pyruvate ¹³C étant détecté, et le signal de l'alanine ¹³C et éventuellement du lactate ¹³C et/ou du pyruvate ¹³C étant utilisé pour générer un profil métabolique indicatif de l'activité d'ALT du foie en cours d'examen, l'information obtenue par le profil métabolique étant utilisée pour surveiller la progression d'une maladie du foie, déterminer la gravité d'une maladie du foie ou identifier des patients à risque de développer une maladie du foie et/ou des candidats de mesures préventives pour éviter le développement d'une maladie du foie aigué ou chronique.

5. Milieu d'imagerie pour l'utilisation selon la revendication 4, dans lequel l'activité d'ALT est déterminée à un premier instant et à un instant ultérieur au cours d'une période de temps.

6. Milieu d'imagerie pour l'utilisation selon la revendication 4, dans lequel le milieu d'imagerie est administré à un corps d'animal humain ou non-humain et une séquence d'imagerie par résonance magnétique est appliquée à moins de 400 secondes après l'administration.

7. Milieu d'imagerie pour l'utilisation selon la revendication 4, dans lequel le pyruvate ¹³C hyperpolarisé est obtenu par une polarisation nucléaire dynamique de l'acide pyruvique ¹³C ou du pyruvate ¹³C.
